Europäisches Patentamt

European Patent Office

Office européen des brevets

(19)

(11) **EP 1 299 719 B1**

(12) **EUROPEAN PATENT SPECIFICATION**

(45) Date of publication and mention
of the grant of the patent:
**03.03.2004 Bulletin 2004/10**

(21) Application number: **01949857.5**

(22) Date of filing: **12.07.2001**

(51) Int Cl.[7]: **G01N 33/493**, G01N 33/88

(86) International application number:
**PCT/IL2001/000642**

(87) International publication number:
**WO 2002/006830 (24.01.2002 Gazette 2002/04)**

(54) **CONDUCTIVITY-NORMALIZED URINARY ANALYTE CONCENTRATION MEASUREMENT FOR USE IN DISEASE DIAGNOSIS**

ANHAND DER KONDUKTIVITÄT NORMALISIERTE BESTIMMUNG VON ANALYTEN IN URIN ZUR DIAGNOSE VON KRANKHEITEN

MESURE DE LA CONCENTRATION D'UN ANALYTE URINAIRE APRES NORMALISATION DE LA CONDUCTIVITE POUR DIAGNOSTIC DE MALADIES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.07.2000 IL 13730800**

(43) Date of publication of application:
**09.04.2003 Bulletin 2003/15**

(73) Proprietor: **Biopreventive Ltd.**
**Migdal Haemek 23103 (IL)**

(72) Inventors:
• **RUBIN, Yoram**
**34987 Haifa (IL)**
• **NIMRI, Shai**
**19150 (IL)**
• **GALILI-NACHSHON, Nitsa**
**18915 D.N. Gilboa (IL)**
• **ALON, Sari**
**20142 (IL)**
• **BEN-TZVI TZHORI, Inbal**
**30063 (IL)**

(74) Representative:
**Weiss, Wolfgang, Dipl.-Chem. Dr. et al**
**Weickmann & Weickmann**
**Patentanwälte**
**Postfach 86 08 20**
**81635 München (DE)**

(56) References cited:
• **TISELIUS H-G: "CALCIUM OXALATE CRYSTALLIZATION PROPERTIES IN URINE WITH DIFFERENT SPECIFIC ELECTRICAL CONDUCTIVITIES" JOURNAL OF UROLOGY, vol. 148, no. 3 PART 2, 1992, pages 990-994, XP002183353 ISSN: 0022-5347**
• **ZHAO Y ET AL: "Urinary Thromboxane B2 in Cardiac Transplant Patients as a Screening Method of Rejection - Heart rejection study group" PROSTAGLANDINS, BUTTERWORTH, STONEHAM, MA, US, vol. 54, no. 6, 1 December 1997 (1997-12-01), pages 881-889, XP004113185 ISSN: 0090-6980**
• **ANDERSEN O ET AL: "SPECIFIC CONDUCTIVITY OF URINE AND SENSITIVITY OF ENZYME IMMUNOASSAY METHODS OF ANALYSIS FOR DRUGS OF ABUSE" CLINICAL CHEMISTRY, vol. 23, no. 4, 1977, pages 751-753, XP002183354 ISSN: 0009-9147**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 299 719 B1

## Description

The present invention is concerned with the use of measurements of urinary electrical conductivity and of the concentrations of relevant urinary analytes in disease diagnosis. More particularly, the present invention relates to the use of conductivity-normalized measurements of urinary thromboxane concentrations in the diagnosis of acute cardiac conditions.

## Background of the Invention

One of the key requirements for the effective management of medical conditions is the availability of accurate, convenient and rapid methods of diagnosis. While this is true of all disease states, it is perhaps most relevant in the case of potentially life-threatening conditions such as acute cardiac disease.

The group of diseases affecting the heart and blood vessels is one of the leading causes of morbidity and mortality. In particular, Acute Coronary Syndrome (ACS) is a leading cause of death in the Western world. While the group of cardiovascular disease taken as a whole consists of a large number of different disease entities, each with it own specific pathogenetic factors, a common element among many of the most prevalent cardiovascular conditions is the formation of athersclerotic plaque, with all its varied biochemical and pathophysiological consequences.

On a worldwide scale, more than 70 million people present at hospitals and other primary health care providers complaining of chest pain each year. In the United States alone, over six million people present with chest pain each year, a statistic that is reflected in the fact that cardiovascular disease accounts for fully one quarter of the current annual health expenditure in the US.

Since the effectiveness of treatment falls exponentially from the time of a myocardial event, the ability to rapidly and accurately diagnose cardiovascular pathology, and thereby commence appropriate treatment at a much earlier stage, is critical in reducing the number of deaths from heart disease.

An additional medical benefit to be derived from improved diagnostic technology screening is the capability to detect patients at risk of developing atherosclerotic lesions and subsequent cardiovascular (and cerebrovascular) pathology. This is of obvious benefit to the development of reliable strategies for the prevention of serious cardiovascular disease.

Finally, the development of early and accurate diagnostic tests will enable health services to reduce the number of unnecessary hospital stays and expensive tests that are administered, providing significant cost savings. Currently, the total annual cost of testing patients for ACS, according to the American College of Cardiology, is estimated to be about $6 billion.

The thromboxanes are compounds derived from prostaglandin endoperoxides that cause platelet aggregation, arterial contraction and many other biological effects. One such compound, thromboxane $A_2$, a highly unstable biologically active bicyclic oxitane-oxane compound, displays very potent vasoconstricting and platelet aggregating properties. thromboxane $A_2$ has been found to play a crucial role in atherothrombotic disorders, and increased synthesis thereof has been found to occur immediately following events such as unstable angina and acute myocardial infarction [Fitzgerald, D.J. *et al*. (1986) N. Engl. J. Med. 315: 983-989]. As mentioned above, thromboxane $A_2$ is very unstable, and is rapidly converted to stable metabolites such as 11-dehydrothromboxane $B_2$ and 2,3, di-northromboxane $B_2$ (collectively referred to hereinbelow as "thromboxane $B_2$"), which are excreted in the urine.

The electrical properties of ion-containing solutions may be expressed in several different ways. One such defining property is that of resistance to electrical current. Electrical resistance (R) may be defined as:

$$R = \rho \times (L/A)$$

wherein L and A are respectively the length and cross-sectional area of the medium whose resistance is being determined, and $\rho$ is the constant of proportionality known as the resistivity of the sample. A commonly used measure of the electrical properties of an electrolytic solution is conductivity, which is defined as the inverse of the resistivity, and is usually measured in units of Siemens per centimeter (S/cm).

The conductivity of urine is, *inter alia*, a measure of urinary dilution, and as such has been used in investigations of the pathogenesis of several different disease states. One such example is the relationship between the degree of urinary dilution and the risk of calcium oxalate crystallization. In one study [Tiselius, H.G. (1992) J. Urol. 148: 990-994], the electrical conductivity of urine was determined in order to assess the degree of urinary dilution. The results of this study suggested that the monitoring of urine dilution by use of a conductivity meter may provide a useful monitoring tool for the prevention of calcium stone disease.

It is a purpose of this invention to provide a urine analysis method for the reliable diagnosis of a variety of medical and surgical conditions.

It is another purpose of the invention to provide such a method that is technically simple to perform, yielding rapid results, thus obviating the need for long waiting periods before appropriate treatment regimes can be initiated.
It is a particular aim of the present invention to provide such a method that may be used in the diagnosis of acute cardiac conditions.
It is a further object of the present invention to provide a diagnostic assay that may be' used very early in the development of cardiac conditions, such that it may be used as an early-warning, first-window technique, yielding reliable predictive data.
It is a further purpose of the invention to provide an assay. for the diagnosis of acute cardiac conditions that overcomes the problems of prior art methods.
Other objects and advantages of the invention will become apparent as the description proceeds.

## SUMMARY OF THE INVENTION

It has now been surprisingly found that the measurement of both the electrical conductivity and the concentration of relevant analytes in urine samples may be used as a reliable diagnostic and predictive tool. Unexpectedly, it was found that the use of conductivity determinations as a means for normalizing urinary analyte concentrations leads to a significant increase in diagnostic reliability and accuracy.
The invention is primarily directed to a method for the diagnosis of a medical condition in a subject, comprising the steps of:

a) obtaining a sample of urine from said subject;
b) measuring the concentration of one or more analytes relevant for the diagnosis of said medical condition in said urine sample;
c) measuring the electrical conductivity of said urine sample;
d) obtaining a normalized value (NTV) for the analyte concentration by dividing the analyte concentration obtained in step b) by the electrical conductivity of the urine sample obtained in step c);
e) determining whether said subject is suffering from said medical condition by means of comparing the NTV obtained in step d) with a pre-determined reference value.

The above-mentioned steps b) and c) may be performed in any order, or alternatively, may be performed simultaneously.
The term cardiac condition as used herein is to be taken to mean pathological conditions of the heart or blood vessels, including atherosclerotic conditions and pathological thrombogenic conditions.
In a preferred embodiment of the method of the invention, the medical condition to be diagnosed is an acute cardiac condition, and the relevant urinary analyte comprises one or more thromboxanes selected from the group consisting of thromboxane $B_2$, 11-dehydrothromboxane $B_2$, 2,3-di-northromboxane $B_2$, and mixtures thereof.
In a preferred embodiment of the method of the invention, the thromboxane measured is thromboxane $B_2$.
Many different assay techniques may be used to perform the measurements required by the method of the invention.. In one preferred embodiment of the invention, both the analyte (e.g. thromboxane) concentration and the electrical conductivity are measured using an amperometric assay. A suitable amperometric assay for use in the method of the present invention is disclosed in co-pending Israeli patent application no. 132410.
In another preferred embodiment, both the analyte (e.g. thromboxane) concentration and the electrical conductivity are measured using a semiconductor-based device.
In yet another preferred embodiment of the invention, the electrical conductivity of the urine sample is measured using a conductivity meter.
In a further preferred embodiment of the method of the invention, the analyte (e.g. thromboxane) concentration is determined using an immunoassay. In a more preferred embodiment, said immunoassay is an enzymeimmunoassay (EIA).
All the above and other characteristics and advantages of the invention will be further understood from the following illustrative and non-limitative examples of preferred embodiments thereof.

## Detailed Description of Preferred Embodiments

The urinary concentration of the relevant analyte, for example thromboxane $B_2$ compounds, may be determined by the use of any suitable quantitative or semi-quantitative technique. Such suitable techniques include, but are not limited to, enzyme linked immunoassays (ELISA), radioimmunoassays (RIA), immunoturbidimetric assays, amperometric assays, dipstick-type assays, and measurements using semiconductor-based devices. All of the aforementioned techniques are extensively described in the art, and well known to the skilled artisan.

Urinary electrical conductivity may be measured by any of the standard techniques known in the art, including the use of amperometric techniques and dedicated conductivity meters. Solid-state semiconductor devices may also be used. Most conveniently, both the urinary thromboxane concentration and the electrical conductivity determination could be obtained simultaneously by the use of separate channels of the semiconductor device. The two, separate, electrical currents thus generated could be analyzed separately and diagnostic results could be produced, in accordance with the data analysis technique that is described in the following Example.

**Example**

**Correlation of thromboxane/conductivity measurements with clinical diagnosis**

Methods

*Subjects and samples:*

A group of 89 patients in the age range 40-70 presenting in the Emergency. Room of a large district hospital were randomly selected for this study. Samples of urine were collected from each of the patients before they were subjected to any diagnostic or treatment procedures. These urine samples were immediately frozen and stored at -20°C for periods of less than one month, prior to being used for the biochemical analyses.

The patients were also asked whether they were currently taking, or had recently been taking, cyclooxygenase inhibitors such as aspirin.

The medical condition of each patient was also assessed 30 days after taking the urine sample, according to the following two criteria:

1. Any cardiac event
2. Free of chest pain

In making the above clinical assessment, the set of patients was divided into the following sub-groups according to the outcome on admission (i.e. on the same day that the urine samples were obtained):

i. **Referral to cardiac care unit/cardiology department**
ii. **Referral to hospital internal medicine department**
iii. **Discharged**
iv. **All patients**

Comparison of the clinical outcome with the result obtained from the biochemical/conductivity analyses (see below in "Data analysis methods") was performed, in order to determine the sensitivity and specificity of said analyses as diagnostic tools.

*Analytical methods:*

1. Thromboxane $B_2$ analysis

The concentrations of thromboxane $B_2$ in the urine samples were measured using a modification of the Biotrak™ system (Amersham International plc, Little Chalfont, Buckinghamshire, England; code RPN 220). The frozen urine samples were thawed and used directly in the thromboxane assay, without any form of pretreatment. Briefly, 50 µl of each sample or thromboxane $B_2$ standard was added in duplicate to the wells of a microtitre plate pre-coated with donkey anti-rabbit IgG. All standard solution dilutions were made in an assay buffer consisting of 0.1M phosphate buffer, pH 7.5 containing 0.9 % sodium chloride and 0.1 % bovine serum albumin. The same buffer was also used in the preparation of the zero standard (i.e. 0 pg thromboxane. $B_2$) and non-specific binding (i.e. buffer-only) wells. The amount of thromboxane $B_2$ added to the standard wells varied between 0.5 and 64 pg per well. Next, 50 µl of rabbit anti-thromboxane $B_2$ antiserum was added to each well (except for the spectrophotometric blank well). Following this, 50 µl of thromboxane $B_2$-horseradish peroxidase conjugate solution was added to each well (except for the blank well), and the plate incubated with shaking at room temperature for one hour. At the end of this incubation period, the contents of each well were aspirated, and each well washed four times with 400 µl wash buffer (0.01M phosphate buffer, pH 7.5, containing 0.05 % Tween 20). Immediately following the final washing step, 150 µl of enzyme substrate (consisting of 3,3', 5,5'-tetramethylbenzidine and hydrogen peroxide) were added to each well. The plate was then incubated with shaking at room temperature for exactly 15 minutes, to allow development of the colored reaction product. The reaction was stopped by the addition of 100 μl of 1M sulphuric acid into each well. Following thorough mixing, and withing 30 minutes of addition of the sulphuric acid, the optical density of each well at 450 nm was determined using a plate reader.

A calibration curve was constructed for the thromboxane $B_2$ standards by plotting the known thromboxane B amount (x-axis) against the percentage of bound antibody (%B/B$_0$). The latter parameter was calculated according to the following relationship:

$$\%B/B_0 = [(\text{thromboxane standard OD - non-specific binding OD})/(B_0 \text{ OD -}$$

$$\text{non-specific binding OD})] \times 100$$

(wherein each OD reading is the average for duplicate wells).

The sample thromboxane $B_2$ amounts for the samples were obtained by reading directly from the calibration curve.

## 2. Conductivity analysis

The electrical conductivity of each of the urine samples was measured using a CyberScan CON100 conductivity meter (Eutech Instruments Pte Ltd., Singapore).

## 3. Normalization of results

A normalized thromboxane $B_2$ concentration value for each sample tested was obtained by dividing said thromboxane concentration (measured in pg/ml) by the conductivity value (measured in mS/cm), either by using simple division or by using a more advanced statistical model. For easier analysis, all values were transformed into their natural logarithms. Thus the normalization of the thromboxane concentrations was achieved by the subtraction of the natural logarithm of the conductivity value from the natural logarithm of the thromboxane concentration.

## Data analysis methods:

The cut-off indicates a value which dictates if the patient condition is pathological or normal. Cut-off was determined according to Receiver Operating characteristic Curves (ROC), which is a plot of the sensitivity (or the true positive value) vs. the false positive value. This analysis optimizes the correlation between the test results and the clinical outcome.

The results of the various analyses described hereinabove were collected and analyzed according to the following two interpretive 'rules'.

**Rule 1** - based on measurement of thromboxane B2 concentration alone, wherein a positive result (i.e. the presence of cardiac disease) is indicated by a natural logarithm-transformed thromboxane value greater than the cut-off value of 5.1 for patients not taking cyclooxygenase inhibiting drugs (e.g. aspirin), or greater than the cut-off value of 4.9, for patients that are taking or have recently taken such drugs.

**Rule 2** - based on determination of **(In thromboxane B2 concentration - In conductivity value)** (i.e. the logarithmic transformation of the ratio of the thromboxane $B_2$ concentration to the conductivity reading), wherein a positive result (i.e. the presence of cardiac disease) is indicated by a result greater than the cut-off value of 3.2, for patients not taking cyclooxygenase inhibiting drugs (e.g. aspirin), or greater than the cut-off value of 2.7, for patients that are taking or have recently taken such drugs.

The cut-off values are the reference values used in the method of the invention. Preferably, such reference values are based on results of diagnostic tests of large groups of patients.

Following analysis of the data according to the above rules, and tabulation of said data, the sensitivity and specificity of each rule was determined according to the following definitions:

$$\text{Sensitivity (\%) = True positive/(False negative + True positive)} \times 100$$

$$\text{Specificity (\%) = True negative/(False positive + True negative)} \times 100$$

Results:

The results comparing the clinical outcome (any cardiac event /free of chest pain) with the laboratory results, as interpreted by each of the two aforementioned rules are given in Table I (Rule 1: without normalization) and Table II (Rule 2: normalized results). For one of the patients, the conductivity could not be determined.

Table I

|  |  | Negative | | Positive | | All | |
|---|---|---|---|---|---|---|---|
|  |  | N | Column % | N | Column % | N | Column % |
| **Outcome on Admission** | **Outcome:72h/30d** |  |  | 10 | (100.0%) | 10 | (100.0%) |
| **Hospit. CCU/Cardiol.** | **Any Cardiac Event** |  |  |  |  |  |  |
|  | **Free of Chest Pain** |  |  | 1 | (100.0%) | 1 | (100.0%) |
|  | **All** |  |  | 11 | (100.0%) | 11 | (100.0%) |
| **Hospital Internal Med.** | **Outcome:72h/30d Any Cardiac Event** | 9 | (29.0%) | 22 | (70.9%) | 31 | (100.0%) |
|  | **Free of Chest Pain** |  |  | 10 | (100.0%) | 10 | (100.0%) |
|  | **All** | 9 | (21.9%) | 32 | (78.0%) | 41 | (100.0%) |
| **Discharged** | **Outcome:72h/30d Any Cardiac Event** | 2 | (12.5%) | 14 | (87.5%) | 16 | (100.0%) |
|  | **Free of Chest Pain** | 6 | (28.5%) | 15 | (71.4%) | 21 | (100.0%) |
|  | **All** | 8 | (21.6%) | 29 | (78.3%) | 37 | (100.0%) |
| **All** | **Outcome:72h/30d Any Cardiac Event** | 11 | (19.2%) | 46 | (80.7%) | 57 | (100.0%) |
|  | **Free of Chest Pain** | 6 | (18.7%) | 26 | (81.2%) | 32 | (100.0%) |
|  | **All** | 17 | (19.1%) | 72 | (80.8%) | 89 | (100.0%) |

Table II

|  |  | Negative | | Positive | | All | |
|---|---|---|---|---|---|---|---|
|  |  | N | Column % | N | Column % | N | Column % |
| **Outcome on Admission** | **Outcome:72h/30d** | 1 | (10.0%) | 9 | (90.0%) | 10 | (100.0%) |
| **Hospital CCU/ Cardiology** | **Any Cardiac Event** |  |  |  |  |  |  |
|  | **Free of Chest Pain** | 1 | (100.0%) |  |  | 1 | (100.0%) |
|  | **All** | 2 | (18.1%) | 9 | (81.8%) | 11 | (100.0%) |
| **Hospital Internal Medicine** | **Outcome:72h/30d Any Cardiac Event** | 12 | (40.0%) | 18 | (60.0%) | 30 | (100.0%) |
|  | **Free of Chest Pain** | 4 | (40.0%) | 6 | (60.0%) | 10 | (100.0%) |
|  | **All** | 16 | (40.0%) | 24 | (60.0%) | 40 | (100.0%) |

Table II   (continued)

| | | Negative | | Positive | | All | |
|---|---|---|---|---|---|---|---|
| | | N | Column % | N | Column % | N | Column % |
| Discharged | Outcome:72h/30d | 3 | (18.7%) | 13 | (81.2%) | 16 | (100.0%) |
| | Any Cardiac Event | | | | | | |
| | Free of Chest Pain | 9 | (42.8%) | 12 | (57.1%) | 21 | (100.0%) |
| | All | 12 | (32.4%) | 25 | (67.5%) | 37 | (100.0%) |
| All | Outcome:72h/30d | 16 | (28.5%) | 40 | (71.4%) | 56 | (100.0%) |
| | Any Cardiac Event | | | | | | |
| | Free of Chest Pain | 14 | (43.7%) | 18 | (56.2%) | 32 | (100.0%) |
| | All | 30 | (34.0%) | 58 | (65.9%) | 88 | (100.0%) |

The sensitivity and specificity of each of the two Rules (i.e. non-normalized and conductivity-normalized thromboxane measurements) for each of the patient groups are shown in Table III (non-normalized data) and Table IV (conductivity-normalized data).

Table III

| | Non-normalized Thromboxane Data | |
|---|---|---|
| Patient Group | Sensitivity % | Specificity % |
| Hospital CCU/Cardiology | 100 | 0 |
| Hospital Internal Medicine | 70.9 | 0 |
| Discharged | 87.5 | 28.5 |
| All | 80.7 | 18.7 |

Table IV

| | Conductivity-normalized Thromboxane Data | |
|---|---|---|
| Patient Group | Sensitivity % | Specificity % |
| Hospital CCU/Cardiology | 90 | 100 |
| Hospital Internal Medicine | 60 | 40 |
| Discharged | 81.2 | 42.8 |
| All | 71.4 | 43.7 |

It may be seen from Tables III and IV that while both interpretive rules yield results having approximately similar sensitivity levels, Rule 2 (that uses conductivity-normalized thromboxane concentrations) gives much improved specificity results. The significance of the higher specificity level of the conductivity-normalized method is that it may be used to determine in a highly reliable manner which of the patients have *not* suffered from an acute cardiac episode, and therefore may be discharged. It is thus concluded that the conductivity-normalized thromboxane $B_2$ concentration data provides a much more reliable tool for predictive use in the diagnosis of acute coronary conditions.

**Claims**

1.   A method for the diagnosis of a medical condition in a subject, comprising the steps of:

   a) providing a sample of urine from said subject;
   b) measuring the concentration of one or more analytes relevant for the diagnosis of said medical condition in said urine sample;
c) measuring the electrical conductivity of said urine sample;
d) obtaining a normalized value (NTV) for the analyte concentration by dividing said analyte concentration obtained in step b) by the electrical conductivity of the urine sample obtained in step c);
e) determining whether said subject is suffering from said medical condition by means of comparing the NTV obtained in step d) with a pre-determined reference value.

2. A method according to claim 1, wherein the medical condition to be diagnosed is an acute cardiac condition, and wherein the relevant urinary analyte comprises one or more thromboxanes selected from the group consisting of thromboxane $B_2$, 11-dehydrothromboxane $B_2$, 2,3-di-northromboxane $B_2$, and mixtures thereof,

3. A method according to claim 2, wherein the thromboxane measured is thromboxane $B_2$.

4. A method according to claim 2, wherein both the thromboxane concentration and the electrical conductivity are measured using an amperometric assay.

5. A method according to claim 2, wherein both the thromboxane concentration and the electrical conductivity are measured using a semiconductor-based device.

6. A method according to claim 2, wherein the electrical conductivity is measured using a conductivity meter.

7. A method according to claim 2, wherein the thromboxane concentration is determined using an immunoassay.

8. A method according to claim 7, wherein the immunoassay is an enzymeimmunoassay.


**Patentansprüche**

1. Verfahren für die Diagnose einer Erkrankung in einem Subjekt umfassend die Schritte:

a) Bereitstellen einer Urinprobe von dem Subjekt;
b) Messen der Konzentration eines oder mehrerer Analyten, die für die Diagnose der Erkrankung relevant sind, in der Urinprobe;
c) Messen der elektrischen Leitfähigkeit der Urinprobe;
d) Erhalten eines normalisierten Wertes (NTV) für die Analytkonzentration durch Teilen der in Schritt b) erhaltenen Analytkonzentration durch die in Schritt c) erhaltene elektrische Leitfähigkeit der Urinprobe;
e) Bestimmen, ob das Subjekt an der Erkrankung leidet durch Vergleichen des in Schritt d) erhaltenen NTV mit einem vorbestimmten Referenzwert.

2. Verfahren nach Anspruch 1, wobei die Erkrankung die diagnostiziert werden soll eine akute Herzerkrankung ist, und wobei der relevante Urinanalyt ein oder mehrere Thromboxane umfasst, ausgewählt aus der Gruppe bestehend aus Thromboxan $B_2$, 11-Dehydrothromboxan $B_2$, 2,3-Di-Northromboxan $B_2$, und Mischungen davon.

3. Verfahren nach Anspruch 2, wobei das gemessene Thromboxan Thromboxan $B_2$ ist.

4. Verfahren nach Anspruch 2, wobei sowohl die Thromboxankonzentration als auch die elektrische Leitfähigkeit unter Verwendung eines amperometrischen Tests gemessen werden.

5. Verfahren nach Anspruch 2, wobei sowohl die Thromboxankonzentration als auch die elektrische Leitfähigkeit unter Verwendung einer Vorrichtung auf Halbleiterbasis gemessen werden.

6. Verfahren nach Anspruch 2, wobei die elektrische Leitfähigkeit unter Verwendung eines Leitfähigkeits-Meßgeräts gemessen wird.

7. Verfahren nach Anspruch 2, wobei die Thromboxankonzentration unter Verwendung eines Immunoassays bestimmt wird.

8. Verfahren nach Anspruch 7, wobei der Immunoassay ein Enzymimmunoassay ist.

**Revendications**

1. Procédé pour diagnostiquer une condition médicale chez un sujet, comprenant les étapes suivantes :

   a) fournir un échantillon d'urine dudit sujet;

   b) mesurer la concentration d'un ou plusieurs analytes pertinents pour le diagnostic de ladite condition médicale dans ledit échantillon d'urine;

   c) mesurer la conductivité électrique dudit échantillon d'urine;

   d) obtenir une valeur normalisée (NTV) pour la concentration d'analyte en divisant ladite concentration d'analyte obtenue à l'étape b) par la conductivité électrique de l'échantillon d'urine obtenue à l'étape c);

   e) déterminer si ledit sujet souffre de ladite condition médicale en comparant la NTV obtenue à l'étape d) avec une valeur de référence prédéterminée.

2. Procédé selon la revendication 1, dans lequel la condition médicale à diagnostiquer est une condition cardiaque aiguë, et dans lequel l'analyte urinaire pertinent comprend un ou plusieurs thromboxanes choisis dans le groupe consistant en thromboxane $B_2$ , 11-déshydrothromboxane $B_2$, 2,3-di-northromboxane $B_2$ et leurs mélanges.

3. Procédé selon la revendication 2, dans lequel le thromboxane mesuré est le thromboxane $B_2$.

4. Procédé selon la revendication 2, dans lequel on mesure tant la concentration en thromboxane que la conductivité électrique par un test ampèremétrique.

5. Procédé selon la revendication 2, dans lequel on mesure tant la concentration en thromboxane que la conductivité électrique en utilisant un dispositif à base de semi-conducteur.

6. Procédé selon la revendication 2, dans lequel la conductivité électrique est mesurée en utilisant un compteur de conductivité.

7. Procédé selon la revendication 2, dans lequel la concentration en thromboxane est déterminée par un dosage immunologique.

8. Procédé selon la revendication 7, dans lequel le dosage immunologique est un dosage immunoenzymatique.